# EUROPEAN PATENT APPLICATION

(11) **EP 4 273 545 A1**
(43) Date of publication of application: **08.11.2023**
(21) Application number: 23171949.3
(22) Date of filing: 05.05.2023
(51) Int. Cl.: G01N 33/00

(54) **SURFACE MODIFIED MATRIX BARRIER FOR A GAS DETECTOR DEVICE**

(30) Priority: 06.05.2022 US 202263338948 P
(71) Applicant: Carrier Corporation, Palm Beach Gardens, FL 33418 (US)
(72) Inventor: EVJU, Jon, Minneapolis, 55438 (US); JARVIS, John, Minneapolis, 55438 (US)
(74) Representative: Dehns

(57) **Abstract**

A gas detector device (10) including a porous matrix barrier (100) disposed between one or more gas sensors (12, 140) and an environment exterior to the gas detector device (10). The porous matrix barrier (100) includes a coating disposed on a matrix member surface, the coating imparting at least one of hydrophobic or oleophobic characteristic to the matrix member surface. The coating reduces the likelihood that a surface member or a pore of the porous matrix barrier will accumulate or become blocked or clogged with dust, dirt, grime, oils, or water.

## Description

Exemplary embodiments pertain to the art of sensors for gaseous compounds, and, more specifically, to mitigating blockages in gas sensors.

Gas sensors have been used in various applications such as process monitoring and control and safety monitoring. As the compounds can also be flammable or explosive, gas detector devices have also been used for leak detection where such compounds are used or manufactured. Various types of sensors have been used or proposed, including but not limited to metal oxide semiconductor (MOS) sensors, non-dispersive infrared detector (NDIR) sensors, pellistor (pelletized resistor) sensors, and electrochemical cells. In some instances, catalytic bead combustible gas sensors are employed to detect the presence of combustible gases or combustible vapors such that a warning of a potentially hazardous condition may be provided.

MOS sensors rely on interaction between gas test components such as hydrocarbons with adsorbed oxygen on the metal oxide semiconductor surface. In the absence of the gas test components, the metal oxide semiconductor adsorbs atmospheric oxygen at the surface, and this adsorbed oxygen captures free electrons from the metal oxide semiconductor material, resulting in a measurable level of base resistance of the semiconductor at a relatively high level. Upon exposure to gas test components the gas test component interacts with the adsorbed oxygen causing gas test component to release free electrons back to the semiconductor material thereby resulting in a measurable decrease in resistance that can be correlated with a measured level of test gas component

The blockage of a gas or gas compound from passing through a porous barrier, for example, a sinter, a filter, a membrane, or a weather shield, in a gas detector device may render these life-saving devices inoperable. Over time the porous barrier may become clogged in-part by dirt, dust, water, grease, or other environmental particles or containments such as soil, soot, clay, geological mineral particulates, air-borne aerosols, and organic particles such as pollen, cellular debris, biological, or particulate condensates present in the air. Consequently, the gas of interest is impeded in entering the sensing chamber, and thus, the ability to monitor/detect the gas of interest is diminished. Although for operational safety there are known methods to detect a blockage of gas flow in a sensor device, the ability to deter or minimize blockage or clogging of a porous barrier may be a complimentary approach.

The lifetime and performance of a gas detector device may be limited by a characteristic of the sensor device to in-part limit the blockage or clogging of a porous sensor matrix. Therefore, there is a need for an improved gas detector device with a porous barrier designed to deter environmental contaminants from accumulating upon a structural member surface of the barrier, and therefore, extend the operational performance of the device.

The present invention provides a gas detector device including a porous matrix barrier disposed between a gas sensor or a semiconductor metal oxide, and an environment exterior to the gas detector device, wherein the porous matrix barrier comprises a coating disposed on a matrix member surface, the coating imparting at least one of hydrophobic or oleophobic characteristic to the matrix member surface.

In addition to one or more of the device features described above, or as an alternative to any of the foregoing embodiments, the porous matrix barrier is a sintered metal or metal oxide, a ceramic, molecular sieve, a metal screen, or matted or woven glass or polymer fibers.

In addition to one or more of the device features described above, or as an alternative to any of the foregoing embodiments, the molecular sieve comprises zeolites, metal organic frameworks, engineered activated carbon, clay, and combinations thereof.

In addition to one or more of the device features described above, or as an alternative to any of the foregoing embodiments, the coating comprises an organo-fluorine group and is derived from a mixture of silane, a hydroxysilane, alkoxysilane, an organosilane, or a mixture thereof, and a fluorosilane, an organofluorosilane, or a mixture thereof.

In addition to one or more of the device features described above, or as an alternative to any of the foregoing embodiments, the coating comprises an organo-fluorine group and is derived from a mixture of tetraethoxysilane, hexamethyldisilane, hexamethyldisilazane, hexamethyldisiloxane, or a mixture thereof, and a fluorine containing gas.

In addition to one or more of the device features described above, or as an alternative to any of the foregoing embodiments, the coating comprises an organo-fluorine group and is derived from a compound of Formula 1:

CₙF₂ₙ₊₁-(CH₂)ₘ-SiR¹R²R³ Formula 1

wherein,
R¹ is a C₁-C₅ alkoxy or CₙF₂ₙ₊₁-(CH₂)ₘ-Si(R²R³)-O-, R² and R³ are independently C₁-C₅ alkyl or C₁-C₅ alkoxy, n is 1 to 12, and m is 1 to 6.

In addition to one or more of the device features described above, or as an alternative to any of the foregoing embodiments, the coating comprises silicone nanoparticles derived from one or more compounds of Formula 2

R⁵Si(R⁶)ₙ(X¹)₃₋ₙ Formula 2:

wherein
R⁵ is a straight-chain or branched C1-C24 alkyl, a C2-C24 alkenyl group, or a C5-C14 aromatic group, which is linked to the silicon atom by a covalent bond or a C1-C8 straight-chain or branched alkylene linker;
R⁶ a straight chain or a branched C1-C6 hydrocarbon group;
X¹ is a hydrolysable group, and is a halogen or a C1-C6 alkoxy group, and n is 0 or 1.

In addition to one or more of the device features described above, or as an alternative to any of the foregoing embodiments, the gas detector includes at least one of the following sensor types, but may include a plurality or combination of the following sensor types: an electrochemical sensor; a MOS sensor, a hot catalytic gas sensor element; a calorimetric sensor, an optical sensor; a photoionization sensor, a photoacoustic sensor; a metal oxide sensor; an acoustic sensor; or a thermal conductivity sensor.

In addition to one or more of the device features described above, or as an alternative to any of the foregoing embodiments, the device further may include a weather shield.

In addition to one or more of the device features described above, or as an alternative to any of the foregoing embodiments, the porous matrix has a thickness of 10 micrometers to 5000 micrometers.

In addition to one or more of the device features described above, the coating further comprises a binder resin, and/or the silicone nanoparticles are present in the form of nanofilaments or nanotubes having a diameter of from 30 nm to 100 nm.

The following descriptions should not be considered limiting in any way. With reference to the accompanying drawings, like elements are numbered alike:
FIG. 1 is a schematic of an embodiment of a gas detector device, in accordance with various aspects of the disclosure; and
FIG. 2 is a schematic of an embodiment of a semiconductor metal oxide type gas detector device, in accordance with various aspects of the disclosure.

A detailed description of one or more embodiments of the disclosed apparatus and method are presented herein by way of exemplification and not limitation with reference to the Figures.

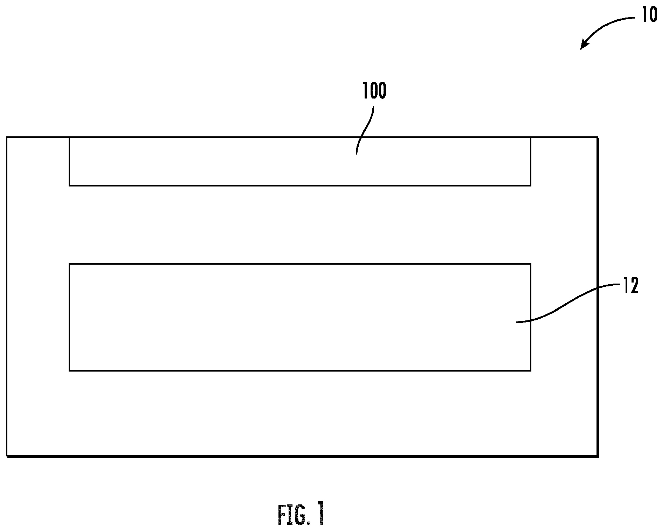

FIG. 1 schematically illustrates an embodiment of a gas detection device 10. The gas detection device 10 may be designed or configured to detect a toxic gaseous compound of interest including, but not limited to, carbon monoxide (CO), hydrogen sulfide (H₂S), ethylene oxide (C₂H₄O), hydrogen fluoride (HF), ammonia (NH₃), sulfur dioxide (SO₂), nitric oxide (NO), nitrogen dioxide (NO₂), or chlorine (Cl₂).

The gas detection device 10 may be designed or configured to detect volatile organic compounds (VOCs) of interest including, but not limited to formaldehyde, chlorinated hydrocarbons, methanol, ethanol, acetone, methyl ethyl ketone, benzene, or toluene. The gas detection device 10 may be designed or configured to detect flammable gases of interest including, but not limited to gases such as hydrogen, ethylene, ethane, propane, methane, liquefied petroleum gas, or other gases.

The gas detection device 10 may be designed or configured to detect refrigerant gases of interest including, but not limited to chlorofluorocarbons (CFCs), hydrochlorofluorocarbons (HCFCs), hydrofluorocarbons (HFCs), A2, A3, or A2L refrigerants.

The gas detection device 10 may be designed or configured to detect gases of interest for indoor air quality monitoring including, but not limited to oxygen (O2), carbon dioxide (CO2), and VOCs. The gas detection device 10 may be designed or configured to detect medically interesting gases of interest during surgical procedures including, but not limited to oxygen (O2), carbon dioxide (CO2), gaseous anesthetics (propofol), nitrous oxide, and the gaseous metabolites of administered anesthesia and medication. The gas detection device 10 may be designed or configured to detect medically interesting gases of interest during diagnostic procedures including, but not limited to specific alcohols, aldehydes, ketones, esters, carboxylic acids, and other gaseous exhalant metabolites, including those from illicit drugs administered pharmaceutical.

The gas detection device 10 may be designed or configured to detect any selection of the gases mentioned above, but not limited to these, in hazardous locations as well as in non-hazardous locations.

The gas detection device 10 includes a sensor 12, e.g., an electrochemical sensor, and a porous matrix barrier 100. The porous matrix barrier 100 forms a protective interface between the gas sensor 12 and the environment exterior to the gas detector device. The porous matrix barrier includes a coating (not shown) disposed on a member surface of the barrier. The coating may reduce the likelihood that a surface member or a pore of the porous matrix barrier will accumulate or become blocked or clogged with dust, dirt, grime, oils, or water. In particular, the coating may impart at least one of hydrophobic or oleophobic characteristic to the member surface of the porous matrix.

In an embodiment, the porous matrix barrier may include at least one of a sintered metal or metal oxide, a ceramic, molecular sieve, a metal screen, or matted or woven glass or polymer fibers. The molecular sieve may include zeolites, metal organic frameworks, engineered activated carbon, clay, and combinations thereof.

We find that a coating with organofluorine functional groups that is covalently bound to the member surface of the porous matrix by siloxane linkages increases surface tension and decrease the affinity for dust, dirt, grime, oil, or water. The coatings are physically and chemically nonreactive, mechanically and structurally stable, and may impart hydrophobic or oleophobic characteristic to the member matrix and may be stable across the UV spectrum.

The coating can be applied to a member surface of a porous matrix by chemical vapor deposition (CVD) including, but not limited to, molecular layer deposition, self-assembled monolayers, ion beam deposition, induced coupled plasma chemical vapor deposition (ICP CVD), microwave plasma chemical vapor deposition, or plasma-enhanced chemical vapor deposition (PECVD). In an embodiment, the coating is applied by PECVD and associated precursors. For instance, in an embodiment, a coating mixture of two or more of silane, a hydroxysilane, an alkoxysilane, an organosilane, a fluorosilane, or an organofluorosilane may be used as coating precursors to provide the coating.

In another embodiment, the coating is applied to by letting organosilane precursors evaporate into a vacuum space containing the parts to be coated. The cyclic application of vacuum and organosilane vapors can be repeated to improve coverage, while excess organosilane precursors can be removed under vacuum.

In another embodiment, the parts to be coated can be dipped in the organosilane precursor solution. A brief vacuum can be applied to evacuate the voids in the porous materials to allow organosilane to enter the pores when the pressure is again increased. The cyclic application of vacuum and organosilane solution can be repeated to improve coverage, while excess silane can be removed under vacuum.

A person of ordinary skill would appreciate that a coating mixture may include other components in addition to any silane-type precursors. For example, the silane precursors may be mixed with water or a solvent mixed with water, an acid or base catalyst, and one or more composition modifying additives to impart or provide a desired chemical structure, properties, function, and performance of the resulting coating. The composition modifying additives may include particle size modifying additives, cross-linking additives, and surface modifying additives.

A person of ordinary skill in the art will recognize that the surfaces to be modified in the porous barrier need to be cleaned and prepared by suitable chemical methods prior to introduction of the silane precursor.

In an embodiment, examples of precursor organosilanes that may be used in a vapor deposition known to those in the coating art may include, but not limited to, tetraethoxysilane (TEOS), hexamethyldisilane (HMDS), hexamethyldisilazane (HMDSN), or desirably hexamethyldisiloxane (HMDSO). Fluorine may be incorporated into the coating using a fluorine containing gas, which may also include carbon, for example, a fluorocarbon including, but not limited to tetrafluoromethane (CF₄), tetrafluoroethylene (C₂F₄), hexafluoroethane (C₂F₆), or trifluoromethane (HCF₃). The fluorine containing gas may comprise silicon, for example: trifluoromethyl trimethylsilane, silicon tetrafluoride. In the case of SiF₄, dihydrogen, SiH₄ or any known organosilane RₓSi_{y}H_{z} may be used to neutralize the fluorine ions, where x is 1 to 8, y is 1 to 4, and z is 1 to 8. A gas such as a noble gas, e.g. argon, neon or helium. Oxygen, nitrogen and/or hydrogen may be used to increase the pressure in the gas and improve the homogeneity of the eventual coating.

In an embodiment, the alkoxysilane may be alkyltrialkoxysilane or tetraalkoxysilane. The alkyl group for alkyltrialkoxysilane can be methyl, ethyl, or propyl. The tetraalkoxysilane is of the type Si(OR)₄ where R=H, methyl, ethyl, isopropyl, or t-butyl or a mixture thereof. The tetraalkoxysilane can be methoxy, ethoxy, or isopropoxy or t-butoxy analogs or a mixture thereof. The organosilane may be of the type R'Si(OR)₃, where R is H, methyl, ethyl, isopropyl, or t-butyl, and R' is methyl, ethyl, or propyl. The organosilane precursor methyltrimethoxysilane can be substituted with triethoxy or tri-isopropoxy derivatives. The organofluorosilane may be of the type Rf'Si(OR)₃ where R is H, methyl, ethyl, isopropyl, or t-butyl, and Rf is 3,3,3-trifluoropropyl, or tridecafluoro-1,1,2,2-tetrahydrooctyl. The organofluorosilane precursor can be (3,3,3-trifluoropropyl)trimethoxysilane or (tridecafluoro-1,1,2,2-tetrahydrooctyl) trimethoxysilane. In some embodiments, the three silane precursors used may include tetramethoxysilane or tetraethoxysilane (as the tetraalkoxysilane), methyltrimethoxysilane (as the organosilane), and trifluoropropyltrimethoxysilane or tridecafluoro-1,1,2,2-tetrahydrooctyl)trimethoxysilane (as the organofluorosilane).

The mole ratio of the silane precursors can be varied independently to fine tune and modify the overall characteristics of the coating. For example, the mole ratio of the silane precursors is important for the stability of the sol as well as for the function and performance of the coating. For example, the relative ratio of the precursors may be important to form a stable cross-linked network for mechanical stability and abrasion resistance. For example, for improved abrasion resistance, the organosilanes and organofluorosilanes are capable of forming three Si--O--Si linkages, while the tetraalkoxysilanes can form four Si--O--Si linkages. Therefore, the relative ratio of each of these precursors is important in forming a stable cross-linked network for mechanical stability and abrasion resistance.

The coating can be applied to a member surface of a porous matrix as a coating composition that includes a silane precursor or a combination of silane precursors that when hydrolyzed and condensed forms a particulate suspension of particles in a liquid sol. The sol can be coated onto a member surface using coating techniques known in the art to form a gel, which is then dried and/or cured to form the coating. For example, in a sol-gel process the silanes are hydrolyzed to silanols that further condense to form siloxane linkages.

The solvent can have an effect on the rate of hydrolysis and condensation of the silane precursors. Also, the particle size and the consequent viscosity of the sol will often depend upon the weight percent of particles and the state of their aggregation in the sol. A solvent that exhibits strong hydrogen bonding and has hydrophilic properties can stabilize individual sol particles to make the sol less viscous and more stable over time. A less viscous sol can form thin films on the order of 50 nm to 500 nm. The boiling point and rate of evaporation of the solvent can also have an effect on formation of a uniform film on the porous matrix. Solvents with relatively low boiling points with an appreciable rate of evaporation are more desirable in forming a uniform film. As the solvent evaporates, the silicate particles come closer together and form a network.

In an embodiment, the coating may be derived from a compound of Formula 1:

CₙF₂ₙ₊₁-(CH₂)ₘ-SiR¹R²R³ Formula 1

wherein,
R¹ is a C₁-C₅ alkoxy or CₙF₂ₙ₊₁-(CH₂)ₘ-Si(R²R³)-O-, R² and R³ are independently C₁-C₅ alkyl or C₁-C₅ alkoxy, n is 1 to 12, and m is 1 to 6.

In an embodiment, the organosilicon compounds of Formula I may include a terminal or extending of a polyfluoroalkyl group with 1 to 12 carbon atoms that is attached to a silicon atom via an alkylene group having 1 to 6 carbon atoms. The radical R¹ can also be a siloxyl group in which one radical is a polyfluoroalkyl group as described above. Examples of typical compounds of the formula I are triethoxy (3,3,4,4,5,5,6,6,7,7,7-undecafluoroheptyl)silane, triethoxy(3,3,4,4,5,5,6,6,7,7,8,8,8-tridecafluorooctyl)silane, triethoxy(3,3,4,4,5,5,6,6,7,7,8,8,9,9,10,10,10-heptadecafluorodecyl)silane, diethoxymetyl(3,3,4,4,5,5,6,6,7,7,8,8,9,9,10,10,10-heptadecafluorodecyl)silane, or bis[ethoxymethyl(3,3,4,4,5,5,6,6,7,7,8,8,8-tridecafluorooctyl)]silyl ether.

The compounds of can be applied to a member surface of the porous matrix using methods known in the art coating including CVD. The compounds can be evaporated readily in a medium high vacuum at temperatures of 25 °C to 120 °C and deposit on a member surface to form relatively thin coatings, and under such deposition conditions the compounds of Formula I are thermally stable. These coatings can subsequently be heated to 160 °C to 200 °C to drive off the low molecular weight condensation products and make the covalent bonds between the porous barrier and the organosilanes.

In an embodiment, the coating may be prepared or derived from a liquid coating composition comprising a solvent and silicone nanoparticles dispersed in the solvent. The silicone nanoparticles can be derived from hydrolysable compound of Formula 2. The silicone nanoparticles may be present in an amount of from 0.01 wt% to 40 wt% based on the total weight of the liquid coating composition. The liquid coating composition is applied to member surface of the porous matrix, and the solvent can be removed by evaporation or drying.

R⁵Si(R⁶)ₙ(X¹)₃₋ₙ Formula 2:

wherein
R⁵ is a straight-chain or branched C1-C24 alkyl, a C2-C24 alkenyl group, or a C5-C14 aromatic group, which is linked to the silicon atom by a covalent bond or a C1-C8 straight-chain or branched alkylene linker;
R⁶ a straight chain or a branched C1-C6 hydrocarbon group;
X¹ is a hydrolysable group, and is a halogen or a C1-C6 alkoxy group, and
n is 0 or 1.

The silicone nanoparticles are formed by polymerization of at least one compound of Formula 2 in an aprotic solvent comprising 5 to 500 ppm, 60 to 250 ppm, or 75 to 150 ppm, of water. A suitable aprotic solvent for the preparation of dispersed silicone nanoparticles in the liquid coating composition can be for example cyclopentane, cyclohexane, 1,4-dioxane, benzene, diethyl ether, chloroform, toluene, dichloromethane, tetrahydrofuran, ethyl acetate, acetone, dimethylformamide acetonitrile, dimethylsulfoxide or mixtures thereof. Preferably, the aprotic solvent is toluene or benzene. However, once the silicone nanoparticles are formed and separated from the polymerization solvent, the nanoparticles can be dispersed in a more environmentally-friendly protic solvent such as a C1-C5 alcohol. Exemplary alcohols are methanol, ethanol, n-propanol, isopropanol, or mixtures thereof. The dispersed silicone nanoparticles may be present in the liquid composition in an amount of from 0.1 wt% to 40 wt%, from 0.1 wt% to 20 wt%, or of from 0.1 wt% to 5 wt%.

In an embodiment, the liquid coating composition may further include a binder resin capable of forming a coating matrix upon evaporation of the solvent. The inclusion of a resin may also allow one to obtain a coating with hydrophobic or oleophobic characteristics in which the dispersed silicone nanoparticles are present on a surface member of the matrix coating. The binder resin may also confer excellent abrasion properties to the coating and may enhance the physical stability of the coating by securing or anchoring the silicone nanoparticles in the coating matrix. Exemplary binder resins include, but not limited to, acrylic, alkyd or phenolic resins, fluorinated resins, polyolefins, polyesters, polyamides, polyurethanes, and mixtures thereof.

The liquid coating composition can be applied to a member surface of the porous matrix by pneumatic atomization of the composition, by dip coating, by electrostatic spraying, or by thermal spraying. In the case where R⁵ is an alkenyl group, for example, the alkenyl group R⁵ includes a terminal vinyl group, the liquid coating composition can be subsequently cured by radiation such as UV by a crosslinking of the vinyl groups to further enhance the attachment of the silicone nanoparticles to the member surface of the porous matrix.

The coating including the silicone nanoparticles may be present in the form of nanofilaments or nanotubes having a diameter of from 30 nm to 100 nm.

In an embodiment, the porous matrix barrier may be a porous, inorganic oxide matrix. The porous, inorganic oxide matrix preferably consists of SiO₂, TiO₂, ZrO₂, MgO, Al₂O₃ or mixtures thereof. The porous, inorganic oxide matrix may be prepared, for instance, by forming a selective shape, e.g., a disc-shaped, of matrix oxide, which is commercially available in finely divided form with particle sizes of between 5 micrometers and 20 micrometers, and then, subjecting the discs to sintering temperatures generally known in the art. For example, the sintering step is typically carried out at temperatures above 700 °C, e.g., 800 °C to 1100 °C, over a period of 1 to 10 hours. Depending on the particle size of the primary particles, on densification and on sintering conditions, the resulting porous sintered articles have a porosity of about 30 vol% to 70 vol%. The sintered articles formed from the porous, inorganic oxide matrix can then be coated with the compounds of the Formula I. The coating may have a thickness of between 20 nanometers to 2 micrometers.

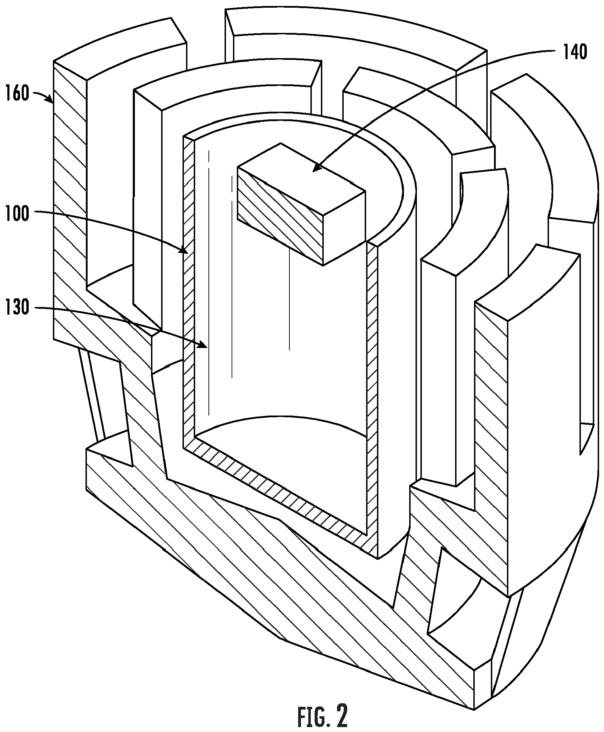

FIG. 2 schematically illustrates a gas detection device according to an embodiment. The device includes a gas sensor 140, a sensing chamber 130, a porous matrix barrier 100, and a weather shield 160. As shown, the porous matrix barrier 100 is disposed between the gas sensor 140 and the exterior environment.

In an embodiment, the weather shield 160 may also include a shield member with a surface coating that imparts at least one of hydrophobic or oleophobic characteristic. As described above, the surface coating may reduce the likelihood that a surface member or a pore of the porous matrix barrier will accumulate or become blocked or clogged with dust, dirt, grime, oils, or water. Moreover, we find that organofluorine functional groups that are covalently bound to the member surface by siloxane linkages increases surface tension and decrease the affinity for dust, dirt, grime, oil, or water. The coatings are physically and chemically nonreactive, mechanically and structurally stable, and may impart hydrophobic or oleophobic characteristic to the member surface and may be stable across the UV spectrum. For instance, in an embodiment, and as described above a coating mixture of two or more of silane, a hydroxysilane, an alkoxysilane, an organosilane, a fluorosilane, or an organofluorosilane may be used as coating precursors to provide the coating.

The sensing chamber 130 behind the porous membrane 100 may contain one, or a plurality of sensors that are the same or different type sensors. The sensing chamber 130 may also contain arrays of sensors.

The porous matrix barrier 100 may be molecular sieve based, polymer based or may be a hybrid filter comprising both molecular sieves and polymer. The porous matrix barrier 100 is designed to prevent or minimize chemical compounds from entering the gas sensing device that may poison the membrane electrode assembly. Exemplary molecular sieves include zeolites, metal organic frameworks, engineered activated carbon, clay, and combinations thereof. Engineered activated carbon is designed and produced to have a specific pore size and thus differs from an adsorbent activated carbon. The pore size of the molecular sieves is chosen based on the size of the molecules that are allowed to pass through as well as the size of the molecules that are to be excluded. For example, the pore size may be chosen to be big enough to allow carbon monoxide through but small enough to exclude compounds such as styrene, cyclohexanone and other cyclic compounds. Exemplary pore sizes include 2 to 10 micrometers.

A hybrid filter may take one of several forms such as a molecular sieve deposited on a polymer layer, a molecular sieve dispersed in a polymer, where the molecular sieve can be dispersed randomly or in layers, or a combination thereof in the filter.

The porous matrix barrier will often have a thickness greater than or equal to 10 micrometers and less than or equal to 5000 micrometers, but the invention is not limited to these thickness dimensions. Within this range the selective membrane may have a thickness greater than or equal to 20 micrometers, or, greater than or equal to 50 micrometers. Also, within this range, the selective membrane may have a thickness less than or equal to 1000 micrometers or less than or equal to 500 micrometers.

In an embodiment, the gas detector device includes a gas-sensing element with a metal oxide semiconductor body including a metal oxide semiconductor material and a transition metal dopant. Examples of metal oxide semiconductors include but are not limited to aluminum (III) oxide, bismuth (III) oxide, cadmium oxide, cerium (IV) oxide, chromium (III) oxide, cobalt (III) oxide, copper (II) oxide, iron (III) oxide, gallium (III) oxide, indium (III) oxide, molybdenum (VI) oxide, niobium (V) oxide, nickel (II) oxide, tantalum (V) oxide, tin (IV) oxide, titanium (IV) oxide, tungsten (VI) oxide, vanadium (5) oxide, zinc (II) oxide or any mixture of two or more metal oxides. Mixed metal oxides (e.g., SnO₂-CuO or other mixed oxides of the above metal oxides) can also be utilized. Transition metal dopants are used to enhance the responsiveness or selectivity of the metal oxide semiconductor to a target gas, e.g., hydrogen sulfide. In some embodiments, the dopant is a group 5 to group 11 transition metal. Examples of transition metal dopants include copper, silver, gold, iron, ruthenium, nickel, platinum, palladium, vanadium, their stable compounds, or a combination thereof.

In an embodiment, a copper-doped tin oxide can be used for hydrogen sulfide sensing elements and platinum and palladium doping is commonly used in sensing for hydrocarbons and hydrogen. Various other materials can be included in the metal oxide semiconductor at the gas-sensing surface 14, including but not limited to noble metals (e.g., silver, gold). Dopants, metal oxide semiconductors, other materials, and combinations thereof are disclosed in Kaur, M. Aswal, D.K. and Yakhmi, J.V."Chemiresistor Gas Sensors: Materials, Mechanisms and Fabrication" Chapter 2 in , Science and Technology of Chemiresistor Gas Sensors, Ed. Aswal, D.K. and Gupta, S.K. Nova Science Publishers, New York, 2007.

In an embodiment, a sensing element can be incorporated into a sensor of a gas detection device. A gas sensor comprises a gas-sensing element with a metal oxide semiconductor body and gas-sensing surface integrated with either parallel or interdigitated electrodes configured to have doped metal oxide semiconductor at the gas-sensing surface disposed between the interdigitated electrodes. The electrodes may be disposed on top of the sensing element but can also be disposed at the bottom. The electrodes are connected externally to the gas-sensing element by an electrical circuit that includes a signal processor. Signal processor can be a voltmeter or ampere meter, but in many cases comprises a potentiostatic circuit, voltage divider circuit, bridge circuit, microprocessor, electronic control unit (ECU), or similar electronic device with integrated voltage and or amperage measurement functions and also can apply a voltage bias between the electrodes. Other sensor components may include, but not limited to, a sensor housing, mounting hardware, gas flow conduits, fluid chambers that are incorporated into the sensor by the skilled person. Additional detail of the gas detector device and the above-described sensing element is described in U.S. Application Serial No. 16/343,156 (US 2019/0317036 A1), assigned to Carrier Corporation, the disclosure of which in its entirety is herein incorporated by reference.

The terminology used herein is for the purpose of describing particular embodiments only and is not intended to be limiting of the present invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof. Furthermore, the terms "comprises" and/or "comprising," as well as the terms "includes" and/or "including," support embodiments which do not incorporate elements other than those described.

While the present invention has been described with reference to an exemplary embodiment or embodiments, it will be understood by those skilled in the art that various changes may be made and equivalents may be substituted for elements of the described embodiment(s) without departing from the scope of the present invention. In addition, many modifications may be made to adapt a particular situation or material to the teachings herein without departing from the scope of the present invention. Therefore, it is intended that the present invention not be limited to the particular embodiment disclosed, but that the present invention will include all embodiments falling within the scope of the claims.

The following clauses set out aspect of the invention which may or may not presently be claimed, but which may provide basis for a future amendment or a divisional application.
1. A gas detector device comprising:
   a porous matrix barrier disposed between one or more gas sensors and an environment exterior to the gas detector device, wherein the porous matrix barrier comprises a coating disposed on a member matrix surface, the coating imparting at least one of hydrophobic or oleophobic characteristic to the member matrix surface.
2. The gas detector device of clause 1, wherein the porous matrix barrier is a sintered metal or metal oxide, a ceramic, a molecular sieve, a metal screen, or matted or woven glass or polymer fibers.
3. The gas detector device of clause 1, wherein the coating comprises an organo-fluorine group and is derived from a mixture of silane, a hydroxysilane, alkoxysilane, an organosilane, or a mixture thereof, and a fluorosilane, an organofluorosilane, or a mixture thereof.
4. The gas detector device of clause 1, wherein the coating comprises an organo-fluorine group and is derived from a mixture of tetraethoxysilane, hexamethyldisilane, hexamethyldisilazane, hexamethyldisiloxane, or a mixture thereof, and a fluorine containing gas.
5. The gas detector device of clause 1, wherein the coating comprises an organo-fluorine group and is derived from a compound of Formula 1:

   CₙF₂ₙ₊₁-(CH₂)ₘ-SiR¹R²R³ Formula 1

   wherein,
   R¹ is a C₁-C₅ alkoxy or CₙF₂ₙ₊₁-(CH₂)ₘ-Si(R²R³)-O-, R² and R³ are independently C₁-C₅ alkyl or C₁-C₅ alkoxy, n is 1 to 12, and m is 1 to 6.
6. The gas detector device of clause 2, wherein the molecular sieve comprises zeolites, metal organic frameworks, engineered activated carbon, clay, and combinations thereof.
7. The gas detector device of clause 1, wherein the gas sensor includes at least one of the following:
   an electrochemical sensor that includes a membrane electrode assembly;
   a MOS sensor;
   a hot catalytic gas sensor element;
   an optical sensor;
   a thermal conductivity sensor;
   a non-dispersive infrared sensor;
   a metal oxide sensor;
   a photoionization sensor; or
   a combination or array of the above chemical sensors.
8. The gas detector device of clause 1, further comprising a weather shield.
9. The gas detector of clause 8, wherein the weather shields includes a shield member with a surface coating that imparts at least one of hydrophobic or oleophobic characteristic.
10. The gas detector device of clause 1, wherein the porous matrix has a thickness of 10 micrometers to 5000 micrometers.
11. The gas detector device of clause 1, wherein the porous matrix has a thickness of greater than 5 millimeters.
12. The gas detector device of clause 1, wherein the coating comprises silicone nanoparticles derived from one or more compounds of Formula 2

   R⁵Si(R⁶)ₙ(X¹)₃₋ₙ Formula 2:

   wherein
   R⁵ is a straight-chain or branched C1-C24 alkyl, a C2-C24 alkenyl group, or a C5-C14 aromatic group, which is linked to the silicon atom by a covalent bond or a C1-C8 straight-chain or branched alkylene linker;
   R⁶ a straight chain or a branched C1-C6 hydrocarbon group;
   X¹ is a hydrolysable group, and is a halogen or a C1-C6 alkoxy group, and
   n is 0 or 1.
13. The gas detector device of clause 12, wherein the silicone nanoparticles are present in the form of nanofilaments or nanotubes having a diameter of from 30 nm to 100 nm.
14. The gas detector device of clause 9, wherein the surface coating of the shield member comprises an organo-fluorine group and is derived from a mixture of silane, a hydroxysilane, an alkoxysilane, an organosilane, or a mixture thereof, and a fluorosilane, an organofluorosilane, or a mixture thereof.
15. The gas detector device of clause 9, wherein the surface coating of the shield member comprises an organo-fluorine group and is derived from a compound of Formula 1:

   CₙF₂ₙ₊₁-(CH₂)ₘ-SiR¹R²R³ Formula 1

   wherein,
   R¹ is a C₁-C₅ alkoxy or CₙF₂ₙ₊₁-(CH₂)ₘ-Si(R²R³)-O-, R² and R³ are independently C₁-C₅ alkyl or C₁-C₅ alkoxy, n is 1 to 12, and m is 1 to 6.
16. The gas detector device of clause 7, wherein the gas sensor is an electrochemical sensor that includes a membrane electrode assembly.
17. The gas detector device of clause 7, wherein the gas sensor is a hot catalytic gas sensor element.
18. The gas detector device of clause 7, wherein the gas sensor is an optical sensor or a thermal conductivity sensor.

## Claims

1. A gas detector device (10) comprising:
a porous matrix barrier (100) disposed between one or more gas sensors (12; 140) and an environment exterior to the gas detector device (10), wherein the porous matrix barrier (100) comprises a coating disposed on a member matrix surface, the coating imparting at least one of hydrophobic or oleophobic characteristic to the member matrix surface.

2. The gas detector device (10) of claim 1, wherein the porous matrix barrier is a sintered metal or metal oxide, a ceramic, a molecular sieve, a metal screen, or matted or woven glass or polymer fibers.

3. The gas detector device (10) of claim 2, wherein the porous matrix barrier (100) is a molecular sieve, and wherein the molecular sieve comprises zeolites, metal organic frameworks, engineered activated carbon, clay, and combinations thereof.

4. The gas detector device (10) of claim 1, 2 or 3, wherein the coating comprises an organo-fluorine group and is derived from a mixture of silane, a hydroxysilane, alkoxysilane, an organosilane, or a mixture thereof, and a fluorosilane, an organofluorosilane, or a mixture thereof.

5. The gas detector device (10) of claim 1, 2 or 3, wherein the coating comprises an organo-fluorine group and is derived from a mixture of tetraethoxysilane, hexamethyldisilane, hexamethyldisilazane, hexamethyldisiloxane, or a mixture thereof, and a fluorine containing gas.

6. The gas detector device (10) of claim 1, 2 or 3, wherein the coating comprises an organo-fluorine group and is derived from a compound of Formula 1:
CₙF₂ₙ₊₁-(CH₂)ₘ-SiR¹R²R³
wherein,
R¹ is a C₁-C₅ alkoxy or CₙF₂ₙ₊₁-(CH₂)ₘ-Si(R²R³)-O-,
R² and R³ are independently C₁-C₅ alkyl or C₁-C₅ alkoxy,
n is 1 to 12, and
m is 1 to 6.

7. The gas detector device (10) of claim 1, 2 or 3, wherein the coating comprises silicone nanoparticles derived from one or more compounds of Formula 2:
R⁵Si(R⁶)ₙ(X¹)₃₋ₙ
wherein
R⁵ is a straight-chain or branched C1-C24 alkyl, a C2-C24 alkenyl group, or a C5-C14 aromatic group, which is linked to the silicon atom by a covalent bond or a C1-C8 straight-chain or branched alkylene linker,
R⁶ a straight chain or a branched C1-C6 hydrocarbon group,
X¹ is a hydrolysable group, and is a halogen or a C1-C6 alkoxy group, and
n is 0 or 1.

8. The gas detector device (10) of claim 7, wherein the silicone nanoparticles are present in the form of nanofilaments or nanotubes having a diameter of from 30 nm to 100 nm.

9. The gas detector device (10) of any preceding claim, further comprising a weather shield (160).

10. The gas detector device (10) of claim 9, wherein the weather shield (160) includes a shield member with a surface coating that imparts at least one of hydrophobic or oleophobic characteristic, and
optionally wherein the surface coating of the shield member comprises an organo-fluorine group and is derived from a mixture of silane, a hydroxysilane, an alkoxysilane, an organosilane, or a mixture thereof, and a fluorosilane, an organofluorosilane, or a mixture thereof.

11. The gas detector device (10) of any preceding claim, wherein the porous matrix (100) has a thickness of 10 micrometers to 5000 micrometers, or wherein the porous matrix (100) has a thickness of greater than 5 millimeters.

12. The gas detector device (10) of any preceding claim, wherein the gas sensor (12; 140) is one of the following:
an electrochemical sensor that includes a membrane electrode assembly;
a MOS sensor;
a hot catalytic gas sensor element;
an optical sensor;
a thermal conductivity sensor;
a non-dispersive infrared sensor;
a metal oxide sensor;
a photoionization sensor; or
a combination or array of the above chemical sensors.

13. The gas detector device (10) of claim 12, wherein the gas sensor (12; 140) is an electrochemical sensor that includes a membrane electrode assembly.

14. The gas detector device (10) of claim 12, wherein the gas sensor (12; 140) is a hot catalytic gas sensor element.

15. The gas detector device (10) of claim 12, wherein the gas sensor (12; 140) is an optical sensor or a thermal conductivity sensor.
